# EUROPEAN PATENT SPECIFICATION

(11) **EP 2 895 075 B1**
(45) Date of publication and mention of the grant of the patent: **13.04.2016**
(21) Application number: 13783393.5
(22) Date of filing: 22.08.2013
(51) Int. Cl.: A61B 8/00, A61B 8/08, A61B 8/14

(54) **MOBILE 3D WIRELESS ULTRASOUND IMAGE ACQUISITION DEVICE AND ULTRASOUND IMAGING SYSTEM**
MOBILE UND DRAHTLOSE 3D-ULTRASCHALL-BILDERFASSUNGSVORRICHTUNG UND ULTRASCHALLBILDGEBUNGSSYSTEM
DISPOSITIF D'ACQUISITION D'IMAGE ULTRASONIQUE SANS FIL 3D MOBILE ET SYSTÈME D'IMAGERIE ULTRASONIQUE

(30) Priority: 13.09.2012 US 201261700471 P
(43) Date of publication of application: 22.07.2015
(73) Proprietor: Koninklijke Philips N.V., 5656 AE Eindhoven (NL)
(72) Inventor: POLAND, McKee Dunn, NL-5656 AE Eindhoven (NL)
(74) Representative: Gravendeel, Cornelis
(86) International application number: PCT/IB2013/056818
(87) International publication number: WO 2014/041448

(56) References cited:
- WO-A1-00/30540
- WO-A1-2008/146209
- US-A1- 2003 139 664
- US-A1- 2011 213 251
- US-B1- 6 440 072

## Description

### FIELD OF THE INVENTION

The present invention relates to an ultrasound image acquisition device for use together with a mobile console to form an ultrasound imaging system. Further, the present invention relates to an ultrasound imaging system for providing an image of a volume, for example an anatomical view within a body of a patient.

### BACKGROUND OF THE INVENTION

Ultrasound imaging systems are widely known in the art. They are in particular used to provide anatomical imaging of views within the body of patients. Both two-dimensional and three-dimensional imaging of bodies of patients is known to provide a reliable tool for medical practitioners to view parts of a body of a patient without the need for any surgical steps.

In three-dimensional ultrasound imaging, or volume imaging, the acquisition of a three-dimensional image may be accomplished by conducting many two-dimensional scans that slice through the volume of interest. Hence, a multitude of two-dimensional images is acquired that lie next to another. By proper image processing, a three-dimensional image of the volume of interest can be built out of the multitude of two-dimensional images. The three-dimensional information acquired from the multitude of two-dimensional images is displayed in proper form on a display for the user of the ultrasound system.

Further, so-called live three-dimensional imaging, or 4D imaging, is often used in clinical applications. In live three-dimensional imaging, a real-time view on the volume can be acquired enabling a user to view moving parts of the anatomical site, for example a beating heart or else.

Ultrasound imaging systems are complete stations that may be fixed to a certain location and are often movable on rollers to provide flexible use in different locations. The ultrasound imaging systems provide for every component needed to acquire ultrasound images, i.e. input devices, display devices, any computer hardware needed to run the ultrasound imaging system and the specific software for acquiring, rendering and displaying the ultrasound images. Further, the ultrasound imaging systems comprise at least one probe carrying one- or two-dimensional transducer arrays to scan the body of a patient either manually or automatically. In order to provide three-dimensional imaging, a probe may utilize a two-dimensional transducer array to electronically steer scan lines in a three-dimensional space. Alternatively, using a one-dimensional transducer array, the array may be scanned manually or automatically by means of a motor to steer scan lines in three-dimensional space.

Of course, providing fully set-up ultrasound imaging systems comprising every component as mentioned above makes these systems not only relatively costly but also large, heavy and inconvenient to move in medical locations.

Further, mobile computational devices are commonly known and have spread throughout clinical applications in the last couple of years. Nowadays, mobile phones, tablets, personal computers and notebooks are largely used to provide all kinds of applications and network access independent of their location. These mobile consoles have steadily increasing hardware performance levels, easy to use interfaces and displays with increasing resolution and quality.

Recent developments have enhanced the functionality of such mobile devices.

Document US 6 440 072 B1 discloses a medical diagnostic ultrasound imaging system and method for transferring ultrasound examination data to a portable computing device. The ultrasound examination data is transferred from a medical diagnostic ultrasound imaging system to a portable computing device, such as a personal digital assistant. The ultrasound examination data can be viewed on the portable computing device or further transferred to a review station or another portable computing device for review. In some preferred embodiments, the examination data is converted from a form readable by the ultrasound system to a form readable by the portable computing device or review station. The ultrasound examination data can be transferred using a wired connection or using wireless technology, such an infrared communications link. The preferred embodiments can also be used with other medical acquisition devices and medical examination data. Examination data can also be transferred from medical networks, such as a medical diagnostic ultrasound imaging network.

Document US 2003/0139664 A1 discloses a segmented handheld medical ultrasound system, wherein ultrasound data such as image data in a video format is wirelessly transmitted to a multi-use display device from a handheld ultrasound device.

There is a need to further improve ultrasound imaging systems in terms of costs, portability and multipurpose functionality.

### SUMMARY OF THE INVENTION

It is an object of the present invention to provide an improved ultrasound image acquisition device and an improved ultrasound imaging system.

The invention is defined by independent claim 1. In a first aspect of the present invention, a three-dimensional ultrasound image acquisition device is provided for use together with a mobile console to form a three-dimensional ultrasound imaging system. The ultrasound image acquisition device comprises a transducer array configured to provide an ultrasound receive signal, an image acquisition hardware assembly having a beam former configured to control the transducer array, and further configured to receive the ultrasound receive signal and to provide an image signal, and a signal processor configured to receive the image signal and to provide image data, and an interface for connecting the mobile console with the ultrasound image acquisition device, wherein the ultrasound image acquisition device comprises a main beam former and a multitude of micro-beam formers.

In a further aspect of the present invention, an ultrasound imaging system is presented for providing an image of a volume, comprising an ultrasound image acquisition device comprising a transducer array configured to provide an ultrasound receive signal, an image acquisition hardware assembly having a beam former configured to control the transducer array, wherein the beam former comprises a main beam former and a multitude of micro-beam formers, and further configured to receive the ultrasound receive signal and to provide an image signal, and a signal processor configured to receive the image signal and to provide image data, and wherein the ultrasound imaging system further comprises a mobile console, wherein the mobile console has a display and a second input device, and an interface for connecting the mobile console with the ultrasound image acquisition device, and wherein the mobile console and the ultrasound image acquisition device are connected via the interface.

The basic idea of the invention is to provide an ultrasound acquisition device capable of three-dimensional ultrasound imaging with just a light cable or wireless connection to a mobile console or host display, where the mobile console itself has no ultrasound specific hardware. The invention relies on encapsulation of three-dimensional ultrasound acquisition hardware in the ultrasound image acquisition device.

A hand-held three-dimensional ultrasound imaging device may provide a breakthrough for many diagnostic applications. With a very light and flexible cable or a wireless connection, ergonomic issues of traditional three-dimensional ultrasound scanners may be overcome. Further, a multiplicity of generic mobile consoles or host devices may be compatible to the ultrasound image acquisition device since the mobile consoles or host devices do not need any ultrasound specific hardware. Mobile live three-dimensional ultrasound imaging is thus enabled. This provides a user with flexibility in customizing and optimizing individual use models of mobile consoles.

Preferred embodiments of the inventions are defined in the dependent claims.

In an embodiment, the three-dimensional ultrasound image acquisition device is a portable probe having a probe housing, and wherein the transducer array and the image acquisition hardware assembly are located within the probe housing. By this, a so-called "smart probe" can be provided. All ultrasound-specific hardware components are located within the probe housing. Further, there is only needed a commercial off-the-shelf (COTS) device as a mobile console to complete a fully functioning ultrasound imaging system. The total power consumption of the probe may be less than 5 W. A probe weight may be less than 200 g. Hence, a flexible system with the mere need to connect the ultrasound acquisition device embodied as a probe to the mobile console of a user can be provided. In particular, the mobile console can be used to command the image acquisition process. A three-dimensional image acquired via the ultrasound image acquisition device can be viewed live and in real time on the mobile console. By providing all ultrasound image acquisition hardware in the probe, the bandwidth of the interface need only be sufficient to transmit image data and display data to the mobile console. Hence, not only single images may be transmitted for storing or display on the mobile console, but a live streaming of the image data and/or display data to the mobile console is enabled. Display data may comprise textual information, such as the user-selected gain level, or graphical data, such as status icons.

In a further embodiment, the transducer array is a phased transducer array, and wherein the interface is a wireless interface. By this, a wireless smart probe can be provided. A wireless probe can connect to a wide variety of commercial off-the-shelf (COTS) computers that include no hardware customization for the ultrasound function, such as tablet or slate computers. This gives the user flexibility in customizing and optimizing individual use models. It also matches the desired ergonomics of a very small handheld mobile console, where the addition of a connector for a cable connecting to the probe would otherwise expand the form factor and create a literal drag on the mobile console acting as a display device, as the mass of the mobile console is approximately as low as that of the probe and cable. With a wireless probe, the handheld console can advantageously be physically separated from the probe, e.g. held, placed on a surface, or mounted on a pole, without the fear that a slight tug from a probe cable while scanning will send the console tumbling.

In an embodiment, the three-dimensional ultrasound image acquisition device further comprises an image processor configured to receive the image data and to provide display data. By this, the three-dimensional ultrasound image acquisition device is enabled to provide for image processing itself. Therefore, even mobile consoles having no significant hardware resources to render three-dimensional images may become compatible for use together with the three-dimensional ultrasound image acquisition device.

In a further embodiment, the ultrasound image acquisition device further comprises a battery powering the ultrasound image acquisition device. By this, the ultrasound image acquisition device can be sufficiently powered while being portable. The term "battery" in this context includes any type of battery, in particular one-way energy cells as well as rechargeable accumulators.

The ultrasound image acquisition device comprises a main beam former and a multitude of micro-beam formers. By this, a possibility of micro-beam forming and cascaded beam forming is provided. By this, the number of signal lines provided via the interface in parallel can be reduced.

In a further embodiment, the wireless interface is further configured for a transmission applying an ultra-wide band transmission technology. By this, sufficient bandwidth for using a mobile console in live three-dimensional ultrasound imaging can be provided. Ultra-wideband (UWB) is a radio technology which operates at a very low energy level for short-range, high-bandwidth communications using a large portion of the radio spectrum. Similar to spread spectrum technology, UWB communications transmit in a way which does not interfere with conventional narrowband and carrier wave used in the same frequency band. Unlike spread spectrum, however, ultra-wideband does not employ frequency-hopping (FHSS). Ultra-wideband is a technology for transmitting information spread over a large bandwidth. UWB can be defined as a transmission from an antenna or interface for which the emitted signal bandwidth exceeds the lesser of 500 MHz or 20% of the center frequency. Thus, pulse-based systems-where each transmitted pulse occupies the UWB bandwidth (or an aggregate of at least 500 MHz of narrow-band carrier; for example, orthogonal frequency-division multiplexing (OFDM)-can gain access to the UWB spectrum. Pulse repetition rates may be either low or very high. Pulse-based UWB radars and imaging systems tend to use low repetition rates (typically in the range of 1 to 100 megapulses per second). On the other hand, communications systems favor high repetition rates (typically in the range of one to two gigapulses per second), thus enabling short-range gigabit-per-second communications systems. Each pulse in a pulse-based UWB system occupies the entire UWB bandwidth (thus reaping the benefits of relative immunity to multipath fading, but not intersymbol interference), unlike carrier-based systems which are subject to deep fading and intersymbol interference. A significant difference between conventional radio transmissions and UWB is that conventional systems transmit information by varying the power level, frequency, and/or phase of a sinusoidal wave. UWB transmissions transmit information by generating radio energy at specific time intervals and occupying a large bandwidth, thus enabling pulse-position or time modulation. The information can also be modulated on UWB signals (pulses) by encoding the polarity of the pulse, its amplitude and/or by using orthogonal pulses. UWB pulses can be sent sporadically at relatively low pulse rates to support time or position modulation, but can also be sent at rates up to the inverse of the UWB pulse bandwidth. Pulse-UWB systems have been demonstrated at channel pulse rates in excess of 1.3 gigapulses per second using a continuous stream of UWB pulses (Continuous Pulse UWB or C-UWB), supporting forward error correction encoded data rates in excess of 675 Mbit/s. Such a pulse-based UWB method (using bursts of pulses) is the basis of the IEEE 802.15.3a draft standard and working group, which has proposed UWB as an alternative PHY layer. Another feature of pulse-based UWB is that the pulses are very short (less than 60 cm for a 500 MHz-wide pulse, less than 23 cm for a 1.3 GHz-bandwidth pulse), so most signal reflections do not overlap the original pulse and the multipath fading of narrowband signals does not exist. However, there is still multipath propagation and inter-pulse interference in fast-pulse systems which must be mitigated by coding techniques. Current working groups and companies working on UWB interfaces and standards are WiMedia Alliance, Bluetooth SIG, Wireless USB, IEEE 802.15.3, IEEE 802.15.3a and IEEE 802.15.4a.

In a further embodiment, the three-dimensional ultrasound image acquisition device has a probe and an intermediate connection device, and wherein the transducer array is located within the probe. In particular, the intermediate connection device is a portable intermediate connection device. By this, a further embodiment may be provided where the image acquisition hardware assembly is located in the intermediate connection device that may be formed as an intermediate box that contains all acquisition hardware. The intermediate connection device may in turn connect to the mobile console, by means of the aforementioned wired or wireless interface. By this, the probe may be designed with an even lower weight and, for example, the intermediate connection device may be positioned at a specific location that provides for good wireless capabilities or an easy to access cable connection port for the mobile console.

In a further embodiment, the signal processor and a main beam former of the beam former are located within the intermediate connection device. Further, the micro beam formers of the beam former are located within the probe. Furthermore, the image processor may be located within the intermediate connection device. Preferably, an intermediate interface between the probe and the intermediate connection device has more than five conductors, preferably fifty conductors plus a power line. By this, probes already known may be used while minimizing size and heat in such probes.

In a further embodiment, the signal processor is located within the intermediate connection device. Further, the image processor may be located within the intermediate connection device. Furthermore, the beam former is located within the probe. In other words, the micro beam formers and the main beam former of the beam former are located within the probe. Preferably, an intermediate interface between the probe and the intermediate connection device has more than five conductors, preferably ten conductors plus a power line. By this, the size of the probe may be reduced, also. Image processor functions and signal processor functions producing heat may still be conducted in the intermediate connection device.

In a further embodiment, the signal processor is located within the probe. Further, the beam former is located within the probe. In other words, the micro beam formers and the main beam former of the beam former are located within the probe. Furthermore, the image processor may be located within the intermediate connection device. Preferably, an intermediate interface between the probe and the intermediate connection device has less than six conductors, preferably four conductors plus a power line. As an alternative, the intermediate interface may be a wireless intermediate interface. By this, the size of the probe may be reduced, also. Image processor functions producing most of the heat dissipated by circuitry may still be conducted in the intermediate connection device.

In a further embodiment, the probe and the intermediate connection device are connected via an intermediate interface, wherein the intermediate interface is a wireless intermediate interface. By this, a sufficient bandwidth can be provided to connect the probe, which may be a micro beam formed probe, to the intermediate connection device and its beam former or master beam former, respectively. In particular, the probe used in connection with the intermediate connection device is a micro beam formed probe.

In a further embodiment, the ultrasound image acquisition device further comprises a first input device for enabling a user to command the ultrasound imaging system. By this, in an embodiment, buttons or the like may be provided on the ultrasound image acquisition device to enable the user to give standard ultrasound image acquisition commands like commands for starting or stopping the acquisition process. Likewise, output indicators such as LEDs may be provided on the ultrasound image acquisition device to show the status of the device or system.

In an embodiment of the ultrasound imaging system, the mobile console has a memory unit having stored thereon an application for viewing the display data on the display of the mobile console. By this, a generic mobile device may be adapted to ultrasound use merely by storing a display software or application (app) on the mobile console. This enables the mobile console to display the data transmitted from the image acquisition device on the display of the mobile console.

In a further embodiment, the mobile console comprises a central processing unit for operating the mobile console, and an image processor configured to receive the image data from the signal processor and to provide display data and a display unit configured to receive the display data and to provide the image. By this, while having sufficient hardware resources, certain processing steps may be conducted by an image processor on the mobile console instead of by its central processing unit or in addition to the central processing unit, for example rendering the image data into a three-dimensional image provided as display data. By this, power consumption and weight of the ultrasound image acquisition device may be further reduced.

In a further embodiment, the ultrasound acquisition device is portable probe having a probe housing, wherein the transducer array and the image acquisition hardware assembly are located within the probe housing, and wherein a weight of the mobile console is less than four times the weight of the portable probe. By this, through use of a wireless interface between the mobile console and the portable probe, use of a COTS mobile console is promoted. The relatively light weight mobile console can be used without any fear that the mobile console will be dragged while using the portable probe or by the weight of a connecting cable.

In a further embodiment, the mobile console is a personal digital assistant (PDA) or a smartphone or a tablet-type personal computer (or slate computer) or a clamshell-type personal computer or a convertible-type personal computer or a hybrid-type personal computer. Slate computers are tablet computers without a dedicated keyboard. For text input, users may rely on handwriting recognition or touching an on-screen keyboard or using an external keyboard that can usually be connected via a wireless or USB connection. Convertible notebooks have a base body with an attached keyboard. They more closely resemble modern laptops, and are usually heavier and larger than slates. Typically, the base of a convertible attaches to the display at a single joint allowing the screen to rotate through 180 degrees and fold down on top of the keyboard. A hybrid-type personal computer shares the features of the slate computer and the convertible-type personal computer by using a detachable keyboard that operates in a similar fashion to a keyboard of a convertible-type personal computer, i.e. rotatable through 180 degrees when attached. By this, a mobile console may be provided as a COTS device readily available in high numbers for any type of clinical personnel.

### BRIEF DESCRIPTION OF THE DRAWINGS

These and other aspects of the invention will be apparent from and elucidated with reference to the embodiment(s) described hereinafter. In the following drawings
Fig. 1 shows a schematic illustration of an embodiment of an ultrasound imaging system,
Fig. 2a shows a schematic block diagram illustrating the processing of signals and data in an ultrasound imaging system and of an ultrasound image acquisition device,
Fig. 2b shows an example of a detailed view on a transducer array and a beam former,
Fig. 3 shows a schematic representation of an ultrasound image acquisition device embodied as a probe,
Fig. 4 shows a schematic block diagram of an embodiment of an ultrasound imaging system,
Fig. 5 shows a block diagram of a further embodiment of an ultrasound imaging system,
Fig. 6 shows a block diagram of another embodiment of an ultrasound imaging system, and
Fig. 7 shows a block diagram of yet another embodiment of an ultrasound imaging system.

### DETAILED DESCRIPTION OF THE INVENTION

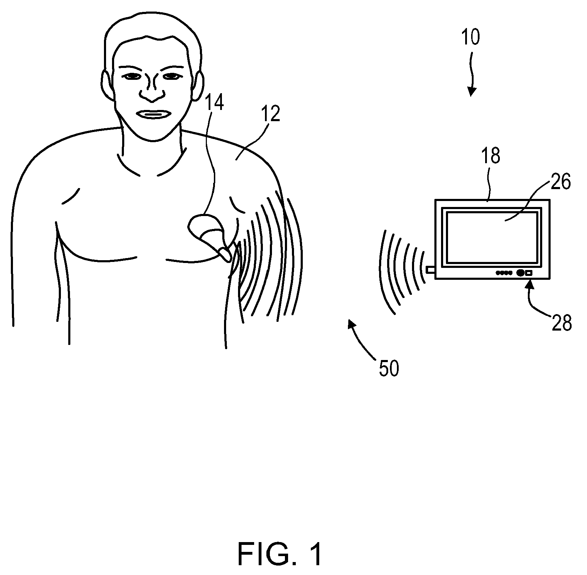

Fig. 1 shows an ultrasound imaging system 10. The ultrasound imaging system 10 is used for scanning an area or volume of the body of the patient 12.

For scanning the patient 12, a probe 14 may be provided. In the embodiment shown, the probe 14 is connected to a mobile console 18. The mobile console 18 is connected to probe 14 via an interface 50 formed in a wireless manner in the embodiment shown in Fig. 1. Further, it is contemplated that the mobile console 18 is connected to the probe 14 in a wireless manner using UWB transmission technology.

The mobile console 18 may comprise a second input device 28. The second input device 28 may have buttons, a keypad and/or a touch screen to provide an input mechanism to a user of the ultrasound imaging system 10. Additionally or alternatively, other mechanisms may be present in the second input device 28 to enable a user to control the ultrasound imaging system 10.

Further, the mobile console 18 comprises a display 26 to display display data generated by the ultrasound imaging system 10 to the user. By this, the volume within the patient 12 that is scanned via the probe 14 can be viewed on the mobile console 18 by the user of the ultrasound imaging system 10.

The "mobile console" 18 may be any computational hardware device that may be carried by a user. In particular, the mobile console 18 may be a cell phone, a PDA (Personal Digital Assistant), a clamshell type personal computer, a tablet type personal computer, a convertible-type personal computer or a hybrid-type personal computer.

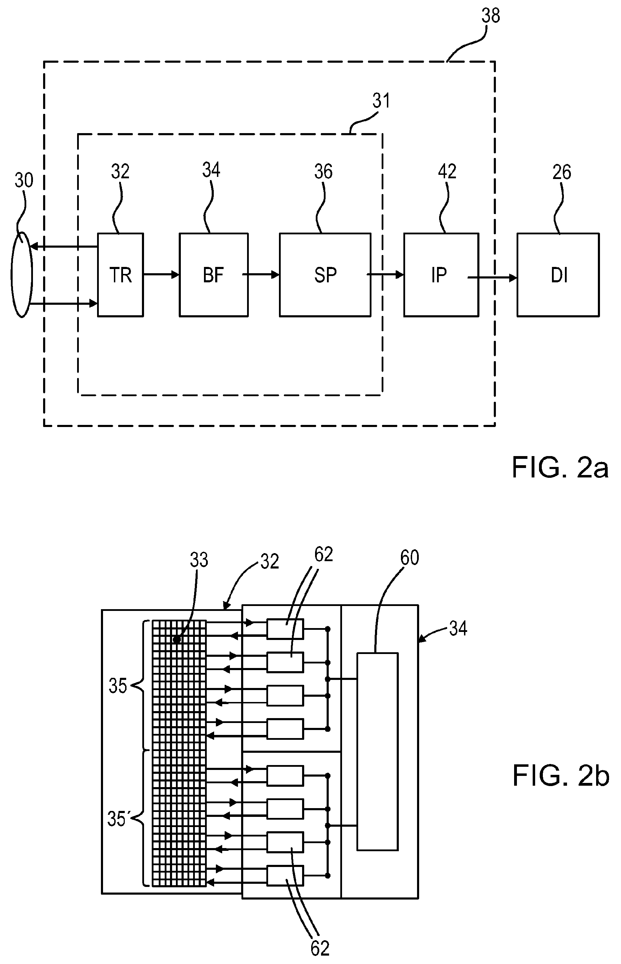

Fig. 2a shows a block diagram illustrating the typical operation of a three-dimensional ultrasound imaging system 10. A transducer array 32 emits ultrasound signals which generate a response from the volume 30 back to the transducer array 32. A beam former 34 explained in more detail blow controls the transducer array 32. The beam former 34 provides an image signal to a signal processor 36. The signal processor 36 in turn generates detected acoustic data - the so-called image data - therefrom. An image processor 42 converts the image data into display data to be displayed on the display 26. The image processor 42 may prepare two-dimensional tomographic slices of the volume 30 to be displayed or may convert or render the image data into a three-dimensional image that is displayed on a display 26.

As initially laid out, the acquisition of a three-dimensional image may be accomplished by conducting many two-dimensional scans that slice through the volume 30. Hence, a multitude of two-dimensional images is acquired that lie next to another with a elevational or rotational displacement. By proper image processing, e.g. shear warp, a three-dimensional image of the volume of interest can be built out of the multitude of two-dimensional images. In case multiple two-dimensional planes are acquired, they may also be displayed side-by-side on the display in a "multi-plane" mode which has significant advantages in particular clinical applications. There are other methods of acquiring voxels, such as by scanning simultaneous quadruplets of receive lines arranged in a rectangular pattern, where the four receive lines utilize simultaneous echoes from a single, centrally placed transmit pulse locus. The quadruplets can be further positioned in any sequence and pattern, including helical.

An image acquisition hardware assembly 31 may be formed by the transducer array 32, the beam former 34 and the signal processor 36. However, the image processor may also be part of the image acquisition hardware assembly 31. This is depicted by the so-called extended image acquisition hardware assembly 38.

Generally, the beam former 32, the signal processor 34 and/or the image processor may be analogue or digitally implemented hardware devices or software implementations run on a processing unit.

Fig. 2b is a schematic detailed view of the transducer array 32 and the beam former 34. The transducer array 32 is formed of a plurality of acoustic elements arranged in a one-dimensional or two-dimensional array. The acoustic elements transmit the ultrasound signals and receive the generated responses. A transducer array 32 may comprise thousands of acoustic elements 33 forming a multitude of sub-arrays 35, 35'. For illustrative purposes, merely two sub-arrays are shown. However, the number of sub-arrays may also be greater than two, e.g. eight. The acoustic elements 33 may, for example, be arranged in a two-dimensional array as a square matrix. However, different shapes such as a rectangular, curved, oval, or circular may also be used, and which is optimal depends mainly on the object being analyzed and the clinical applications.

The transducer array 32 has a plurality of micro beam formers 62, which control both transmission and reception of acoustic pulses through the acoustic elements, and combine the acoustic responses generated by the scanned medium in order to form the sub-array summed acoustic signals, which are then transferred from the transducer array 32 through signal lines to the beam former 34. Shown are two groups of each having four micro beam formers 62. However, the number of micro beam formers 62 in each group may also be different from four, e.g. eight or sixteen. In particular, eight groups each having sixteen micro beam formers 62 may be present. Each signal line within a sub-array 35, 35' may emanate from one micro beam former 62 and is joined with the other signals of that sub-array 35, 35' to form a sub-array group output. The sub-array group output is then connected to the main beam former 60 as described below.

There are two main phases of beam forming, namely, transmit and receive. During transmit, acoustic pulses are generated from acoustic elements of the transducer array 32. During the receive phase, echoes from those pulses in the volume 30 are received by the acoustic elements of the transducer array 32, amplified, and combined. For beam forming in the transmit phase, transmit delay pulsers generate delayed high voltage pulses. The acoustic pulses are transmitted by the acoustic elements. The acoustic pulses are timed relative to each other to generate a focus in the three-dimensional space of the insonified medium. In the receive phase, the acoustic pulses previously transmitted are echoed by structures in the volume 30. Between the time that the acoustic pulses are transmitted and the generated pulses are received by the acoustic elements, so-called T/R (transmit/receive) switches switch to the receive position. Acoustic pulses are received by the acoustic elements from many points on the body, and receive samplers take periodic samples of the resulting acoustic wave to generate analog samples, which are small voltages. The analog samples are then delayed by receive delays. The receive delays may be static delays, meaning they are unchanged during the course of acoustic reception. The receive delays may also be programmable and thereby modified dynamically during the receive phase so as to maintain a constant array focus as the echoes propagate into the medium. The separately delayed received signals are summed together by summers, and after summing, variable gain amplifiers perform time gain compensation. Time variable gain is required because the signals received by the acoustic elements from later times correspond to deeper depths of the body, and are therefore attenuated. The variable gain amplifiers compensate for this attenuation by increasing output. The sub-array summed acoustic signals are transmitted by the signal lines.

Hence, the transducer array 32 provides dynamic or static beamforming to generate a plurality of sub-array summed acoustic signals, which are received by a further static or dynamic beam former in a main beam former 60. The main beam former 60 performs static or dynamic beamforming to generate a set of fully beam formed image signals. Hence, in the current application, the "beam former" 34 denotes the so-called master beam former which is comprised of the micro beam formers 62 and the main beam former 60. Hence, one main beam former 60 sub-groups a multitude of micro beam formers 62. By this, the number of signals from the beam former 34 to the signal processor 36 may be significantly reduced.

Examples of such transducer arrays with cascaded beam forming may be the X6-1 or X7-2 type probes commercialized by the applicant.

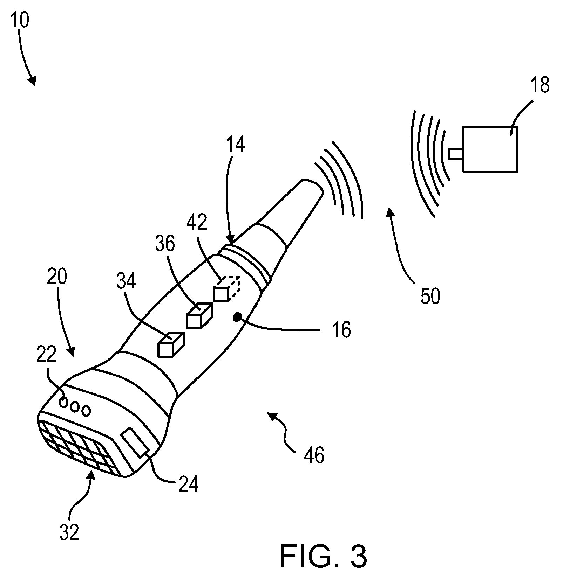

Fig. 3 shows an embodiment wherein the ultrasound image acquisition device 46 that is solely embodied as the probe 14. The probe 14 has a probe housing 16 that includes all necessary ultrasound imaging hardware, that is the transducer array 32, the beam former 34, the signal processor 36 and, optionally, the image processor 42. Further, the probe housing 16 may have a first input device 20 having, for example, a button 22 to control the image acquisition. Further, an output device 22 may be provided at the probe, e.g. in the form of a light emitting diode (LED) or a plurality of lights or LEDs 22. The probe 14 is connected via an interface 50 to the mobile console 18. The embodiment shown in Fig. 3, the interface 50 is a wireless connection. Mobile live three-dimensional ultrasound imaging is thus enabled. This provides a user with flexibility in customizing and optimizing individual use models of the mobile console 18.

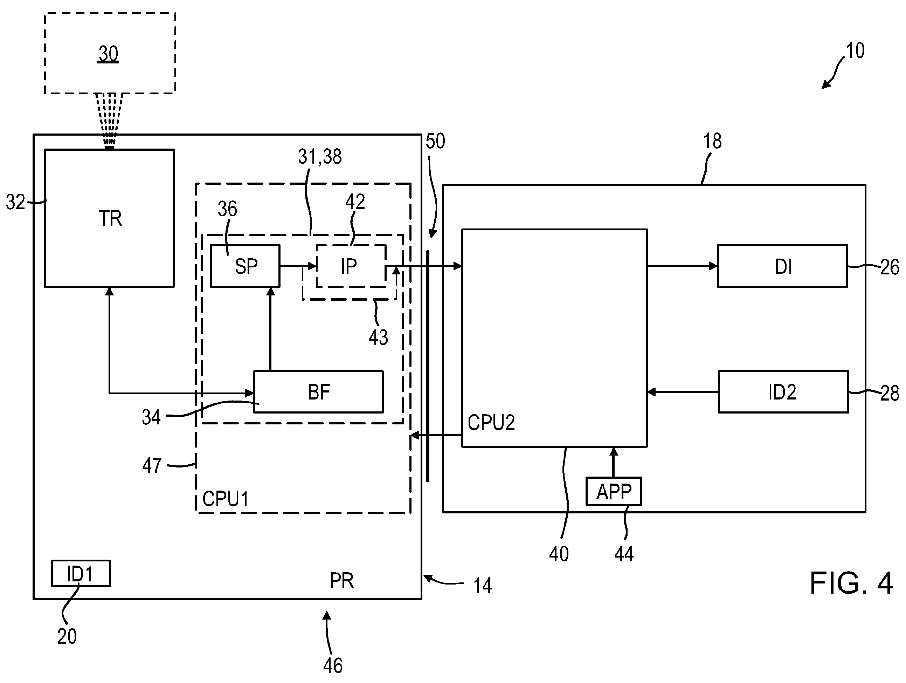

Fig. 4 shows a schematic block diagram as an example for the various components of the ultrasound imaging system 10 and their location and interaction within the whole ultrasound imaging system 10.

As already explained above, the ultrasound imaging system 10 is used for scanning a volume of a patient 12. The volume is schematically shown in dashed lines and designated with reference numeral 30. The area is examined via the probe 14 carrying a transducer array 32. The transducer array 32 may be of any known type. Hence, the transducer array 32 may be a one-dimensional transducer array or a two-dimensional transducer array that may be mechanically or electronically scanned. The transducer array 32 converts the ultrasound signals into electronic signals and vice versa.

To command the transducer array 32, the beam former 34 is present that is used to control the electronic and/or mechanical scanning of the transducer array and, if possible, the number, density and position of scan lines along which the area 30 is scanned. Further, the signal processor 36 may be provided that receives the ultrasound image signal of the beam former and provides image data. The beam former 34 and the signal processor 36 together may form an image acquisition hardware assembly 31.

The image processor 42 receives image data from the signal processor 36 and provides display data to the display 26. The beam former 34, the signal processor 36 and the image processor 42 may be run by the central processing unit 47. In an embodiment, the signal processor 36 and/or the image processor 42 may be of a software-implemented type and run on the central processing unit 47 of the probe 14. However, it may also be the case that at least one or two of the group of the signal processor 36, the beam former 34 and the image processor 42 is/are of a hardware-implemented type, the location of the respective circuitry is preferably as shown in Fig. 4.

The probe 14 comprises, therefore, all necessary ultrasound acquisition hardware in the form of an ultrasound image acquisition hardware assembly 31. The image processor 42 is merely optional inside the probe 14. It may alternatively be provided by the mobile console and its central processing unit 40. Hence, the image processor 42 in Fig. 4 is merely depicted in dashed lines. If not present, the signal processor 36 forwards this data directly to the central processing unit 40 of the mobile console 18 as indicated via the dashed line 43. Further, instead of a software implementation into this central processing unit 40 of the mobile console 18, the image processor 42 may also be hardware implemented in the mobile console 18. The software implemented image processor 42 may also be part of an application 44 run on the central processing unit 40 of the mobile console to provide display data for display on the display device 26.

However, an extended image acquisition hardware assembly 38 may be formed in the probe 14 if the image processor 42 is also present in the probe. The probe 14 may comprise a central processing unit 47 controlling one or more operations of the probe 14. Hence, the signal processor 36 and/or the image processor 42 (if present) may be software implemented and run on the central processing unit 47 of the probe 14. However, the signal processor 36 and/or the image processor 42 may also be hardware implemented in the probe 14 for efficiency or as an application specific security. The first input device 20 of the probe 14 may, in any embodiment, be used to provide simple control of the image acquisition process, like a button to start and stop the image acquisition process.

As apparent from Fig. 4, the mobile console 18 does not need any specific ultrasound image acquisition hardware. An input device as the second input device 28, a display as the display 26 and a central processing unit as the central processing unit 40 are frequently present on any mobile console that is commercially available of the shelf. A specific software or app 44 may then be downloadable or start on the mobile console 18 and run on the central processing unit 40 to view the display data with the rendered images of the volume 30. The operating system stored on the mobile console 18 may for example be a Windows operating system, an Android operating system or an iPhone iOS operating system.

In an embodiment, the transducer 32 is a two-dimensional phased-array matrix-type transducer array which is electronically scanned and micro beam formed to a plurality of channel signals which are further beam formed and demodulated inside the probe 14. Then, as the interface 50, an ultra wideband (UWB) interface is used to connect the probe 14 to the mobile console 18.

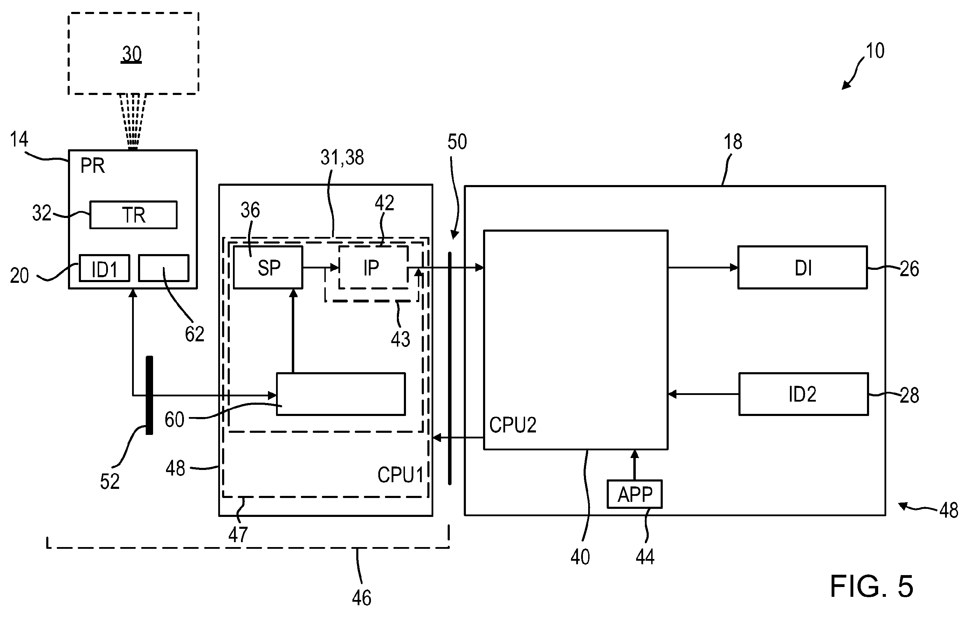

Fig. 5 shows a further embodiment of an ultrasound imaging system 10. Like elements are denoted with like reference numerals and will not be explained again. This embodiment also provides for the advantage that the mobile console 18 does not need to comprise any ultrasound specific hardware. Again, a display 26, an input device 28 and a central processing unit 40 in which an application 44 is run to display the display data on the display device 26 is sufficient. Also, the interface 50 may be as previously explained, it may be cable connected or implemented wirelessly.

However, in this embodiment, the image acquisition device 46 is not solely implemented in the probe 14. Instead, the probe carries the transducer array 32, the micro beam formers 62 and, optionally, a first input device 20. Further, there is provided an intermediate connection device 48 as part of the image acquisition device 46 that is connected via an intermediate interface 52 with the probe 14. In particular, the intermediate connection device 48 can be portable. The intermediate interface 52 may be a cable connection. However, preferably it is also implemented wirelessly. In this embodiment, if the interface 52 is a wireless interface, also the UWB technology may be used to connect the micro beam formers 62 of the transducer 32 to the main beam former 60 . In case the intermediate interface is cable-connected, the interface 52 may also include a power line to power the probe 14 and the intermediate connection device 48 may include a battery for powering both the intermediate connection device 48 and the transducer array 32. In case the intermediate interface is cable-connected, the probe 14 and the intermediate connection device 48 are each powered by a battery. In that case the same battery may also provide power to both the intermediate connection device 48 and probe 14.

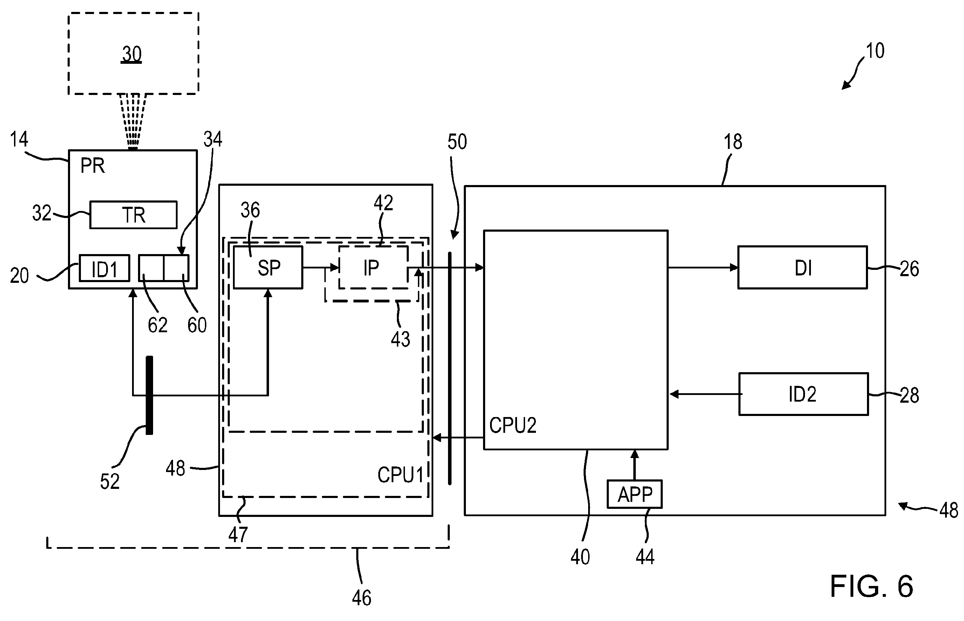

Fig. 6 shows a further embodiment similar to that of Fig. 5. Like elements are designated with like reference numerals and will not be explained again. In this embodiment, the probe 14 also comprises the main beam former 60 and, hence, the whole beam former 34. By this, the size of the probe 14 may be reduced, also. The signal processor 36 is located in the intermediate connection device 48. The image processor 42 may still be located in the intermediate connection device 48. Alternatively, it may be located in the mobile console 18.

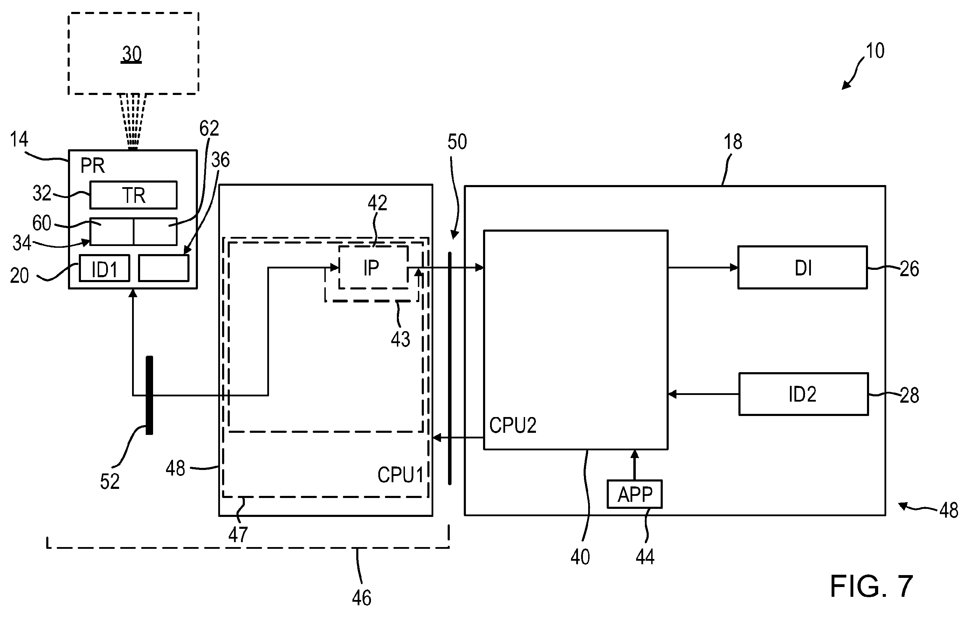

Fig. 7 shows a further embodiment similar to that of Fig. 6. Like elements are designated with like reference numerals and will not be explained again. In this embodiment, the probe 14 also comprises the signal processor 36. By this, the size of the probe may be reduced, also. The image processor 42 producing most of the heat dissipated by its circuitry may still be located in the intermediate connection device 48.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A three-dimensional ultrasound image acquisition device (46) for use together with a mobile console (18) to form a three-dimensional ultrasound imaging system (10), the ultrasound image acquisition device (46) comprising:
a transducer array (32) configured to provide an ultrasound receive signal,
an image acquisition hardware assembly (31) having a beam former (34) configured to control the transducer array (32), and further configured to receive the ultrasound receive signal and to provide an image signal, and a signal processor (36) configured to receive the image signal and to provide image data,
an interface (50) for connecting the mobile console (18) with the ultrasound image acquisition device (46), **characterised in that** the beam former (34) is a master beam former comprised of a main beam former (60) and a multitude of micro beam formers (62), wherein the main beam former (60) is configured to sub-group the multitude of micro beam formers (62).

2. The three-dimensional ultrasound image acquisition device (46) of claim 1, wherein the ultrasound acquisition device (46) is a portable probe (14) having a probe housing (16), and wherein the transducer array (32) and the image acquisition hardware assembly (31) are located within the probe housing (16).

3. The three-dimensional ultrasound image acquisition device (46) of claim 1, wherein the transducer array (32) is a phased transducer array, and wherein the interface (50) is a wireless interface.

4. The three-dimensional ultrasound image acquisition device (46) of claim 1, wherein the three-dimensional ultrasound image acquisition device (46) further comprises an image processor (42) configured to receive the image data and to provide display data.

5. The three-dimensional ultrasound image acquisition device (46) of claim 1, wherein the ultrasound image acquisition device (46) further comprises a battery powering the ultrasound image acquisition device (46).

6. The three-dimensional ultrasound image acquisition device (46) of claim 3, wherein the wireless interface (50) is further configured for transmission applying an ultra-wide band transmission technology.

7. The three-dimensional ultrasound image acquisition device (46) of claim 1, wherein the three-dimensional ultrasound image acquisition device (46) further has a portable probe (14) and an intermediate connection device (48), and wherein the transducer array (32) is located within the probe (14).

8. The three-dimensional ultrasound image acquisition device (46) of claim 7, wherein the main beam former (60) is connected to each of the micro beam formers (62) and wherein the main beam former (60) is connected to the signal processor (36) being located in the intermediate connection device (48).

9. The three-dimensional ultrasound image acquisition device (46) of claim 7, wherein the probe (14) and the intermediate connection device (48) are connected via an intermediate interface (52), wherein the intermediate interface (52) is a wireless intermediate interface.

10. The three-dimensional ultrasound image acquisition device (46) according to claim 1, wherein the ultrasound image acquisition device (46) further comprises a first input device (20) for enabling a user to command the ultrasound imaging system (10).

11. An ultrasound imaging system (10) for providing an three-dimensional image of a volume (30), comprising the ultrasound image acquisition device (46) according to claim 1 and a mobile console (18), wherein the mobile console (18) has a display (26) and a second input device (28), and wherein the mobile console (18) and the ultrasound image acquisition device (46) are connected via the interface (50).

12. The ultrasound imaging system (10) of claim 12, wherein the mobile console (18) has a memory unit (44) having stored thereon an application for viewing the display data on the display (26) of the mobile console (18).

13. The ultrasound imaging system (10) according to claim 11, wherein the mobile console (18) comprises a central processing unit (40) for operating the mobile console (18), and an image processor (42) configured to receive the image data from the signal processor (36) and to provide display data, and a display unit (26) configured to receive the display data and to provide the image.

14. The ultrasound imaging system (10) according to claim 11, wherein the ultrasound acquisition device (46) is a portable probe (14) having a probe housing (16), wherein the transducer array (32) and the image acquisition hardware assembly (31) are located within the probe housing (16), and wherein a weight of the mobile console (18) is less than four times the weight of the portable probe (14).

## Patentansprüche

1. Dreidimensionale Ultraschallbildaufnahmeeinrichtung (46) zum Einsatz zusammen mit einer mobilen Konsole (18), um ein dreidimensionales Ultraschallbildgebungssystem (10) zu bilden, wobei die Ultraschallbildaufnahmeeinrichtung (46) umfasst:
ein Transducerarray (32), das so eingerichtet ist, dass es ein Ultraschallempfangssignal vorsieht,
eine Bildaufnahmehardwareanordnung (31) mit einem Beamformer (34), der so eingerichtet ist, dass er das Transducerarray (32) steuert, und weiterhin so eingerichtet ist, dass er das Ultraschallempfangssignal empfängt und ein Bildsignal vorsieht, sowie mit einem Signalprozessor (36), der so eingerichtet ist, dass er das Bildsignal empfängt und Bilddaten bereitstellt,
eine Schnittstelle (50), um die mobile Konsole (18) mit der Ultraschallbildaufnahmeeinrichtung (46) zu verbinden,
**dadurch gekennzeichnet, dass**
der Beamformer (34) ein Master-Beamformer ist, der sich aus einem Hauptbeamformer (60) und mehreren Mikrobeamformern (62) zusammensetzt, wobei der Hauptbeamformer (60) so eingerichtet ist, dass er die mehreren Mikrobeamformer (62) untergruppiert.

2. Dreidimensionale Ultraschallbildaufnahmeeinrichtung (46) nach Anspruch 1, wobei die Ultraschallaufnahmeeinrichtung (46) eine portable Sonde (14) mit einem Sondengehäuse (16) ist, und wobei das Transducerarray (32) und die Bildaufnahmehardwareanordnung (31) innerhalb des Sondengehäuses (16) positioniert sind.

3. Dreidimensionale Ultraschallbildaufnahmeeinrichtung (46) nach Anspruch 1, wobei das Transducerarray (32) ein phasengesteuertes Transducerarray ist, und wobei die Schnittstelle (50) eine drahtlose Schnittstelle ist.

4. Dreidimensionale Ultraschallbildaufnahmeeinrichtung (46) nach Anspruch 1, wobei die dreidimensionale Ultraschallbildaufnahmeeinrichtung (46) weiterhin einen Bildprozessor (42) umfasst, der so eingerichtet ist, dass er die Bilddaten empfängt und Displaydaten bereitstellt.

5. Dreidimensionale Ultraschallbildaufnahmeeinrichtung (46) nach Anspruch 1, wobei die Ultraschallbildaufnahmeeinrichtung (46) weiterhin eine, die Ultraschallbildaufnahmeeinrichtung (46) speisende Batterie umfasst.

6. Dreidimensionale Ultraschallbildaufnahmeeinrichtung (46) nach Anspruch 3, wobei die drahtlose Schnittstelle (50) weiterhin zur Übertragung unter Anwendung einer Ultra-Breitband-Übertragungstechnologie eingerichtet ist.

7. Dreidimensionale Ultraschallbildaufnahmeeinrichtung (46) nach Anspruch 1, wobei die dreidimensionale Ultraschallbildaufnahmeeinrichtung (46) weiterhin eine portable Sonde (14) und eine Zwischenanschlusseinrichtung (48) aufweist, und wobei das Transducerarray (32) innerhalb der Sonde (14) positioniert ist.

8. Dreidimensionale Ultraschallbildaufnahmeeinrichtung (46) nach Anspruch 7, wobei der Hauptbeamformer (60) mit jedem der Mikrobeamformer (62) verbunden ist, und wobei der Hauptbeamformer (60) mit dem in der Zwischenanschlusseinrichtung (48) positionierten Signalprozessor (36) verbunden ist.

9. Dreidimensionale Ultraschallbildaufnahmeeinrichtung (46) nach Anspruch 7, wobei die Sonde (14) und die Zwischenanschlusseinrichtung (48) über eine Zwischenschnittstelle (52) verbunden sind, wobei die Zwischenschnittstelle (52) eine drahtlose Zwischenschnittstelle ist.

10. Dreidimensionale Ultraschallbildaufnahmeeinrichtung (46) nach Anspruch 1, wobei die Ultraschallbildaufnahmeeinrichtung (46) weiterhin eine erste Eingabeeinrichtung (20) umfasst, über die ein Benutzer das Ultraschallbildgebungssystem (10) anweisen kann.

11. Ultraschallbildgebungssystem (10) zur Bereitstellung eines dreidimensionalen Bildes eines Volumens (30), umfassend die Ultraschallbildaufnahmeeinrichtung (46) nach Anspruch 1 und eine mobile Konsole (18), wobei die mobile Konsole (18) ein Display (26) und eine zweite Eingabeeinrichtung (28) aufweist, und wobei die mobile Konsole (18) und die Ultraschallbildaufnahmeeinrichtung (46) über die Schnittstelle (50) verbunden sind.

12. Ultraschallbildgebungssystem (10) nach Anspruch 11, wobei die mobile Konsole (18) eine Speichereinheit (44) aufweist, auf der eine Applikation zur Darstellung der Displaydaten auf dem Display (26) der mobilen Konsole (18) gespeichert ist.

13. Ultraschallbildgebungssystem (10) nach Anspruch 11, wobei die mobile Konsole (18) eine zentrale Prozessoreinheit (40), um die mobile Konsole (18) und einen Bildprozessor (42) zu betreiben, der so eingerichtet ist, dass er die Bilddaten von dem Signalprozessor (36) empfängt und Displaydaten bereitstellt, sowie eine Displayeinheit (26) umfasst, die so eingerichtet ist, dass sie die Displaydaten empfängt und das Bild bereitstellt.

14. Ultraschallbildgebungssystem (10) nach Anspruch 11, wobei die Ultraschallaufnahmeeinrichtung (46) eine portable Sonde (14) mit einem Sondengehäuse (16) ist, wobei das Transducerarray (32) und die Bildaufnahmehardwareanordnung (31) innerhalb des Sondengehäuses (16) positioniert sind, und wobei ein Gewicht der mobilen Konsole (18) weniger als viermal das Gewicht der portablen Sonde (14) ausmacht.

## Revendications

1. Dispositif d'acquisition d'images ultrasoniques tridimensionnelles (46) pour utilisation conjointement avec une console mobile (18) pour former un système d'imagerie ultrasonique tridimensionnelle (10), le dispositif d'acquisition d'images ultrasoniques (46) comprenant :
un réseau de transducteurs (32) configuré pour fournir un signal de réception ultrasonique,
un ensemble de matériels d'acquisition d'images (31) ayant un formateur de faisceau (34) configuré pour commander le réseau de transducteurs (32) et encore configuré pour recevoir le signal de réception ultrasonique et fournir un signal d'image, et un processeur de signaux (36) configuré pour recevoir le signal d'image et fournir des données d'image,
une interface (50) pour connecter la console mobile (18) au dispositif d'acquisition d'images ultrasoniques (46), **caractérisé en ce que**
le formateur de faisceau (34) est un formateur de faisceau maître constitué d'un formateur de faisceaux principal (60) et d'une pluralité de micro-formateurs de faisceau (62), dans lequel le formateur de faisceau principal (60) est configuré pour sous-grouper la pluralité de micro-formateurs de faisceau (62).

2. Dispositif d'acquisition d'images ultrasoniques tridimensionnelles (46) selon la revendication 1, dans lequel le dispositif d'acquisition ultrasonique (46) est une sonde portable (14) ayant un logement de sonde (16) et dans lequel le réseau de transducteurs (32) et l'ensemble de matériels d'acquisition d'images (31) sont disposés dans le logement de sonde (16).

3. Dispositif d'acquisition d'images ultrasoniques tridimensionnelles (46) selon la revendication 1, dans lequel le réseau de transducteurs (32) est un réseau de transducteurs en phase et dans lequel l'interface (50) est une interface sans fil.

4. Dispositif d'acquisition d'images ultrasoniques tridimensionnelles (46) selon la revendication 1, dans lequel le dispositif d'acquisition d'images ultrasoniques tridimensionnelles (46) comprend en outre un processeur d'images (42) configuré pour recevoir les données d'images et fournir des données d'affichage.

5. Dispositif d'acquisition d'images ultrasoniques tridimensionnelles (46) selon la revendication 1, dans lequel le dispositif d'acquisition d'images ultrasoniques (46) comprend en outre une pile alimentant le dispositif d'acquisition d'images ultrasoniques (46).

6. Dispositif d'acquisition d'images ultrasoniques tridimensionnelles (46) selon la revendication 3, dans lequel l'interface sans fil (50) est en outre configurée pour une transmission en appliquant une technologie de transmission à bande ultralarge.

7. Dispositif d'acquisition d'images ultrasoniques tridimensionnelles (46) selon la revendication 1, dans lequel le dispositif d'acquisition d'images ultrasoniques tridimensionnelles (46) comporte en outre une sonde portable (14) et un dispositif de connexion intermédiaire (48) et dans lequel le réseau de transducteurs (32) est situé dans la sonde (14).

8. Dispositif d'acquisition d'images ultrasoniques tridimensionnelles (46) selon la revendication 7, dans lequel le formateur de faisceau principal (60) est connecté à chacun des micro-formateurs de faisceau (62) et dans lequel le formateur de faisceau principal (60) est connecté au processeur de signaux (36) qui est situé dans le dispositif de connexion intermédiaire (48).

9. Dispositif d'acquisition d'images ultrasoniques tridimensionnelles (46) selon la revendication 7, dans lequel la sonde (14) et le dispositif de connexion intermédiaire (48) sont connectés via une interface intermédiaire (52), dans lequel l'interface intermédiaire (52) est une interface intermédiaire sans fil.

10. Dispositif d'acquisition d'images ultrasoniques tridimensionnelles (46) selon la revendication 1, dans lequel le dispositif d'acquisition d'images ultrasoniques (46) comprend en outre un premier dispositif d'entrée (20) pour permettre à un utilisateur de commander le système d'imagerie ultrasonique (10).

11. Système d'imagerie ultrasonique (10) pour fournir une image tridimensionnelle d'un volume (30), comprenant le dispositif d'acquisition d'images ultrasoniques (46) selon la revendication 1 et une console mobile (18), dans lequel la console mobile (18) a un affichage (26) et un second dispositif d'entrée (28) et dans lequel la console mobile (18) et le dispositif d'acquisition d'images ultrasoniques (46) sont connectés via l'interface (50).

12. Système d'imagerie ultrasonique (10) selon la revendication 12, dans lequel la console mobile (18) a une unité de mémoire (44) dans laquelle est stockée une application pour observer les données d'affichage sur l'affichage (26) de la console mobile (18).

13. Système d'imagerie ultrasonique (10) selon la revendication 11, dans lequel la console mobile (18) comprend une unité de traitement centrale (40) pour faire fonctionner la console mobile (18) et un processeur d'images (42) configuré pour recevoir les données d'images du processeur de signaux (36) et pour fournir des données d'affichage, et une unité d'affichage (26) configurée pour recevoir les données d'affichage et fournir l'image.

14. Système d'imagerie ultrasonique (10) selon la revendication 11, dans lequel le dispositif d'acquisition ultrasonique (46) est une sonde portable (14) ayant un logement de sonde (16), dans lequel le réseau de transducteurs (32) et l'ensemble de matériels d'acquisition d'images (31) sont situés dans le logement de sonde (16) et dans lequel le poids de la console mobile (18) est inférieure à quatre fois le poids de la sonde portable (14).
